# EUROPÄISCHE PATENTSCHRIFT

(11) **EP 2 613 844 B1**
(45) Veröffentlichungstag und Bekanntmachung des Hinweises auf die Patenterteilung: **03.03.2021**
(21) Anmeldenummer: 11770338.9
(22) Anmeldetag: 07.09.2011
(51) Int. Cl.: A61M 35/00, A61M 11/00, A61J 3/00, A61K 9/00

(54) **PERKUTANES APPLIKATIONSSYSTEM**
PERCUTANEOUS APPLICATION SYSTEM
SYSTÈME D'APPLICATION PERCUTANÉ

(30) Priorität: 08.09.2010 DE 102010044674
(43) Veröffentlichungstag der Anmeldung: 17.07.2013
(73) Patentinhaber: MedDrop Technology AG, 8008 Zürich (CH)
(72) Erfinder: TESLENKO, Alexander, 58095 Hagen (DE); GULIK, Dieter, 20257 Hamburg (DE); VON HAHN, Friedrich, 22299 Hamburg (DE)
(74) Vertreter: Schöneborn, Holger
(86) Internationale Anmeldenummer: PCT/EP2011/004497
(87) Internationale Veröffentlichungsnummer: WO 2012/031747

(56) Entgegenhaltungen:
- GB-A- 2 419 532
- US-A1- 2006 041 248
- US-A1- 2009 234 269
- US-A1- 2010 152 880
- US-B1- 6 461 361

## Beschreibung

Die Offenbarung betrifft ein Applikationssystem zur flächigen, transepidermalen Verabreichung pharmazeutischer oder kosmetischer Wirkstoffe, mit einem Behälter zur Aufnahme einer einen Wirkstoff enthaltenden, versprühbaren Zusammensetzung, einer Treibgasquelle für ein unter Druck stehendes Gas, das zu mindestens 25 Vol.-% aus Sauerstoff besteht, und einer Sprühvorrichtung, wobei Behälter, Treibgasquelle und Sprühvorrichtung so miteinander in Strömungsverbindung stehen, dass bei Betätigung der Sprühvorrichtung das Treibgas die versprühbare Zusammensetzung zerstäubt und aus der Sprühvorrichtung heraustreibt.

Die Haut ist das größte Organ des Menschen. Unter anderem dient sie als Barriere gegen das Eindringen von Noxen, insbesondere auch von Krankheitserregern aus der Umgebung. Anatomisch betrachtet ist die Haut ein mehrschichtiges Organ. Der Aufbau ist nach außen begrenzt durch die Epidermis (Oberhaut), einem blutgefäßefreien, verhornenden, mehrschichtigen Plattenepithel mit einer äußeren Hornschicht (Stratum corneum). Darunter liegt die durchblutete Dermis (Lederhaut). Unter der Haut findet sich die Subcutis (Unterhaut-Fettgewebe). In die Haut eingelagert sind Schweißdrüsen, Talgdrüsen und Haare. Die Barrierefunktion der Haut wird vor allem durch die Epidermis bewirkt.

Innerhalb der äußeren Schicht, d. h. der Epidermis (Oberhaut), ist das Stratum corneum von besonderer Bedeutung. Gemäß dem Hautmodell von Elias ähnelt die Hornschicht im Hinblick auf ihren Aufbau einer Ziegelsteinmauer (Ziegelstein-Mörtel-Model). In diesem Modell entsprechen die Hornzellen (Corneozyten) den Ziegelsteinen und die Lipidmembran im Interzellularraum dem Mörtel. Die abgestorbenen Hornzellen schilfern ständig nach außen ab, so dass sich die Hornschicht in einem ständigen Erneuerungsprozess befindet.

Insgesamt stellt die Hornschicht eine wirkungsvolle Barriere gegen das Eindringen von Fremdstoffen, Bakterien, Viren etc. dar. Im Hinblick auf den transdermalen Transport von Arzneistoffen erweist sich die Barrierefunktion der Hautschicht allerdings als hinderlich. Übliche kosmetische und pharmazeutische Zubereitungen vermögen die Epidermisbarriere kaum zu überwinden, eine Penetration der Wirkstoffe findet fast nicht statt.

Bei der systemischen Gabe von Arzneimitteln treten häufig unerwünschte Nebenwirkungen auf. Gerade bei der Verabreichung von Entzündungshemmern (Antiphlogistika) und Schmerzmitteln (Analgetika) etc. über den Gastrointestinaltrakt gibt es nicht selten gravierende Komplikationen. Darreichungsformen, die den Magen-Darm-Trakt umgehen, sind Injektionen, Infusionen etc., die eine Verletzung (Einstich) von Körpergewebe erfordern, oder verletzungsfreie Verfahren wie transdermale Pflaster, sublinguale Arzneimittel usw. mit niedriger Aufnahmerate.

Aus diesen Gründen ist eine topische Verabreichung von Arzneimitteln über die Haut sinnvoll, aber wegen der Barrierefunktion der äußeren Haut bislang nicht einfach durchführbar.

Um die Hautbarriere zu überwinden sind verschiedene Verfahren bekannt, u. a. Elektroporation, Elektrophorese, Sonophorese, transdermale Pflaster oder Jet-Injektionen. Diese Verfahren vermögen aber nur gelöste Wirkstoffe durch die Haut zu transportieren, eine Versorgung der Haut mit Sauerstoff ist nicht möglich oder wurde bisher nicht versucht.

Ein Verfahren gemäß dem Oberbegriff von Anspruch 1, das auf Elektroporation beruht, wird in der US 2009/0234269 A1 beschrieben. Hierbei werden geladene Tröpfchen erzeugt, die ihre Ladung beim Auftreffen auf die Hautoberfläche abgeben, wodurch ein Eintritt durch die Hautbarriere ermöglicht wird.

Von einem therapeutischen Standpunkt aus ist der Erfolg einer transepidermalen Verabreichung von Wirkstoffen für eine lokale Wirkung davon abhängig, inwieweit es gelingt, in der Haut ein optimales Konzentrations-ZeitProfil des Wirkstoffs zu erreichen. Dieser Prozess wird maßgeblich durch die strukturellen und funktionellen Veränderungen der behandelten Haut bestimmt.

Penetration bezeichnet ein Eindringen von Wirkstoffen in die Haut, üblicherweise wird darunter ein Eindringen in die Oberhaut verstanden. Eine Permeation ist dagegen eine Aufnahme durch die Epidermis hindurch in einem Ausmaß, die eine systemische Resorption durch Aufnahme in das Blutgefäßsystem der Dermis ermöglicht. Im medizinischen Bereich sind die eine Permeation beschreibenden Adjektive transdermal oder perkutan, im kosmetischen Bereich wird aber auch die Penetration in die Epidermis so bezeichnet. Diese Begriffe sind deshalb nicht eindeutig. Nachfolgend wird deshalb der Begriff transepidermal verwendet, um eine Permeation von der Hautoberfläche bis in die Dermis zu beschreiben.

Im kosmetischen Bereich sind Sprühsysteme für die Applikation von Zubereitungen auf die Haut bekannt, allgemein üblich und weit verbreitet. Die Barrierefunktion der Haut verhindert jedoch den transepidermalen Transport.

Eine Hypoxie ist ein pathologischer Zustand des gesamten Körpers (generalisierte Hypoxie) oder einer Körperregion (Gewebe-Hypoxie), hervorgerufen durch eine nicht hinreichende Versorgung mit molekularem Sauerstoff. Ein zelluläres Missverhältnis zwischen Sauerstoffversorgung und Sauerstoffbedarf resultiert in einer Hypoxie. Dadurch kommt es zu einer verminderten Energiegewinnung in den Mitochondrien. Diese Umstände führen zur Schädigung der Zellen, was sich als entzündliche Reaktion der betroffenen Gewebe äußert.

Eine anhaltende, chronische Hypoxie ist für die Manifestation mehrerer Erkrankungen verantwortlich (rheumatische und Herz-Kreislauf-Erkrankungen, chronische Wunden, Hirnleistungsschwäche, diabetische Folgekrankheiten etc). Andererseits kann auch eine reaktive Entzündung durch die Verstärkung des Metabolismus zu einer lokalen Hypoxie führen.

Eine therapeutische Versorgung der hypoxischen Zellen und Organe mit Sauerstoff könnte die Hypoxie vermindern oder kompensieren. Auch eine (bspw. medikamentöse) Verstärkung der Durchblutung verbessert die Versorgung mit Sauerstoff.

Die üblichen Sauerstofftherapien basieren auf Inhalationen, sind also systemische Verabreichungen. Darüber hinaus sind aus dem Stand der Technik weitere Formen der Sauerstofftherapie bekannt, beispielsweise die Verabreichung von gasförmigem Sauerstoff, die hyperbare Sauerstofftherapie (HBO) die Verwendung von künstlichem Blut aus Perfluorcarbonen oder die Sauerstoffbegasung bei der Wundbehandlung, hierbei wird jedoch der Sauerstoff nicht über die Haut appliziert. Daneben sind kosmetische Zubereitungen bekannt, welche Sauerstoff enthalten (WO 2005/016309 A1 oder WO 2006/094550 A1) sowie Sprühsysteme, bei denen eine mit Sauerstoff angereicherte Flüssigkeit auf die Haut gesprüht wird und erst anschließend ein Überdruck erzeugt wird, um Wirkstoffe in die Haut einzubringen (US 2005/0228338 A1), die Menge des in die Haut eindringenden Sauerstoffs ist bei diesem Verfahren aber sehr gering. Darüber hinaus kann stets nur ein kleines Hautareal behandelt werden. Derzeit gibt es kein Verfahren, Sauerstoff in ausreichenden Mengen durch die äußere Haut in tiefere Schichten der Haut zu applizieren.

Es ist von großer therapeutischer Bedeutung, ein Verfahren zu finden, das in der Lage ist, einen simultanen Transport von Wirkstoffen und Sauerstoff durch die Haut zu ermöglichen. Bei einer lokalen Behandlung ist es wünschenswert, den Arzneistoff und den Sauerstoff direkt an den zu therapierenden Ort zu bringen, um unerwünschte systemische Nebenwirkungen der Wirkstoffe zu minimieren.

In der WO 2006/040119 A2 wurde bereits ein Verfahren beschrieben, wonach, allerdings ohne weitere Nachweise, die Einbringung von Sauerstoff in die Hornschicht und somit die Erhöhung des Sauerstoffpartialdrucks auf der Gewebeseite des Stratum corneums zu einem verbesserten transdermalen Transport von Arzneistoffen führen soll. Die Arzneistoffe wurden hierbei in Form von Mikroemulsionen verabreicht. Als Arzneistoffe kamen natürliche und künstliche Wirkstoffe unterschiedlichster Art für unterschiedlichste Indikationen in Frage. Verabreicht wurde die Mikroemulsion mit Hilfe eines Applikationssystems, das über eine Vorrichtung zum Zerstäuben der Mikroemulsion mit Hilfe eines Gases, insbesondere Sauerstoffs, verfügt. Allerdings war die Anwendung auf Mikroemulsionen und vergleichsweise kleine Wirkstoffmoleküle beschränkt.

Ausgehend von diesem Stand der Technik stellt sich die Aufgabe, ein Applikationssystem zur perkutanen Verabreichung von Arzneimitteln zur Verfügung zu stellen, mit dem die Wirkstoffe noch wirksamer und großflächiger in die Haut eingebracht werden können, ohne dabei die Haut zu verletzen.

Diese Aufgabe wird erfindungsgemäß gelöst durch ein Applikationssystem nach Anspruch 1.

Überraschend hat sich herausgestellt, dass die Applikation von Wirkstoffen über die Haut sich deutlich verbessern lässt, wenn die Austrittsgeschwindigkeit des Trägergasstromes, welcher die zerstäubte Wirkstoffzusammensetzung trägt, auf mehr als 100 m/s erhöht wird. Bei Verwendung entsprechender Düsen als Sprühvorrichtung können auch größere Moleküle durch die Haut eingebracht werden. Die Wirkstoffzusammensetzung muss nicht unbedingt als Mikroemulsion vorliegen, auch wenn Mikroemulsionen im Hinblick auf die Sauerstofflösung bevorzugt sind, durch die Erhöhung der Austrittsgeschwindigkeit ist jedoch auch die Verabreichung von Emulsionen, Nanoemulsionen, Gelen, Liposomen, Mikrobläschen, Mikroschäumen und sonstigen versprühbaren Zusammensetzungen möglich.

Ein weiterer überraschender Effekt, der sich bei Verwendung einer Düse mit hoher Austrittsgeschwindigkeit, insbesondere einer Laval-Düse, ergeben hat, ist, dass sich erheblich größere Mengen von Sauerstoff in dem Vehikel (der versprühbaren Zusammensetzung) lösen. Dies ermöglicht, zusätzlich zu den Wirkstoffen eine signifikante Menge an Sauerstoff in die Haut einzubringen. Auf diese Weise wird die Therapie einer bspw. entzündlichen lokalen Hypoxie ermöglicht und die Wirkung der verabreichten Wirkstoffe synergistisch verstärkt.

Düsen, die eine Austrittsgeschwindigkeit von mehr als 100 m/s aufweisen, sind zwar auch aus der Jet-Technologie bekannt, bei der eine Flüssigkeit mechanisch oder auch mit Hilfe eines Treibgases kurzzeitig (explosionsartig) auf hohe Geschwindigkeiten beschleunigt wird, bei dieser Technologie wird jedoch die Haut mit einem scharfen Strahl durchdrungen und entsprechend verletzt. Letztlich handelt es sich bei der Jet-Technologie um eine Alternative zu herkömmlichen Injektionen mit Hilfe einer Nadel. Entsprechend wird die Jet-Technologie vorwiegend für systemische Verabreichungen, beispielsweise Impfungen verwendet.

Im Gegensatz dazu wird bei dem erfindungsgemäßen System die Wirkstoffzusammensetzung großflächig über einen längeren Zeitraum von mehreren Minuten kontinuierlich verabreicht. Der Sprühstrahl tritt kegelförmig aus. Durch Bewegung der Austrittsdüse können dabei große Hautareale behandelt werden. Die Behandlung kann über einen längeren Zeitraum von 10 bis 30 min erfolgen. Der Austrittswinkel aus der Sprühvorrichtung, d. h. der Öffnungswinkel des Sprühstrahlkegels, beträgt mindestens 10°, vorzugsweise mindestens 15°, besonders bevorzugt mindestens 20°. Typisch sind Werte zwischen 15° und 30°. Denkbar sind jedoch auch noch größere Austrittswinkel, um eine große Oberfläche zu besprühen, ohne dass die Sprühvorrichtung selbst bewegt werden muss, aus praktischen Gründen beträgt der Austrittswinkel jedoch in der Regel nicht mehr als 45° oder 60°. Auch bei kleinerem Sprühstrahlkegel lassen sich jedoch große Hautoberflächen besprühen, da die Sprühvorrichtung in Bewegung gehalten wird. Die besprühte Oberfläche beträgt meist 10 bis 100 cm², kann jedoch, insbesondere bei veterinärmedizinischen Anwendungen, auch noch deutlich größer sein. Die Haut wird dabei nicht verletzt oder auf sonstige Weise in Anspruch genommen, vielmehr führt allein die Sauerstoffanreicherung unter der Haut zu einer temporären Überwindung der Barrierefunktion der Haut verbunden mit einem transdermalen Transport der Wirkstoffe.

Das Verhältnis von Länge zu Durchmesser des Austritts der Sprühvorrichtung hat einen Einfluss auf die Zusammensetzung des Sprühstrahls. Ein relativ kurz gehaltener Austritt erzeugt tendenziell einen größeren Strahlkegelwinkel (Austrittswinkel) als ein in Strömungsrichtung längerer Austritt. Dies führt zu einer größeren vom Sprühstrahl erreichten Oberfläche bei gleichzeitiger Abnahme der Strahleindringtiefe.

Die Erfindung unterscheidet sich von der bekannten Jet-Technologie nicht nur dadurch, dass die zu applizierende Wirkstoffzusammensetzung großflächig auf die Haut aufgebracht wird, sondern auch durch die Verwendung von Sauerstoff als Treibgas. Im Rahmen der Jet-Technik werden hingegen andere, inerte Gase verwendet (Stickstoff, Kohlendioxid, Argon), die Verabreichung von Sauerstoff über die Haut spielt hierbei keine Rolle. Ein solches Verfahren wird beispielsweise in der US 2006/0041248 A1 beschrieben; hier handelt es sich um eine nadellose Injektion unter Verwendung von überkritischen Flüssigkeiten, insbesondere überkritischem CO₂. Auch hier werden hohe Geschwindigkeiten des Sprühstrahls erzielt, darüber hinaus kann die Düse für einen mehr oder weniger stark divergierenden Sprühstrahl eingestellt werden. Die Verwendung großer Mengen sauerstoffhaltigen Treibgases oder die Verwendung eines Turbinenrads spielen hier jedoch keine Rolle.

Der beim erfindungsgemäßen Applikationssystem verwendete Sauerstoff findet nicht nur als Trägergas Verwendung, sondern wird als ein weiterer "Wirkstoff" mit appliziert. Es hat sich überraschend gezeigt, dass bei der Behandlung verschiedener Krankheiten eine synergistische Wirkung des Wirkstoffs selbst und des Sauerstoffs zum Tragen kommt. Hypoxisches Gewebe, d. h. Gewebe mit einem herabgesetzten Sauerstoffgehalt, steht im Zusammenhang mit einer Reihe von Erkrankungen. Eine Sauerstoffunterversorgung wird insbesondere durch eine nicht ausreichende Gewebedurchblutung (mangelnde Mikrozirkulation) oder durch eine zu niedrige O₂-Bindungskapazität von Hämoglobin herbeigeführt. Erkrankungen, die mit einem hypoxischen Zustand in Zusammenhang stehen, sind beispielsweise Rheuma oder Psoriasis.

Durch die Verwendung einer anderen Sprühvorrichtung, bei der die Austrittsgeschwindigkeit mindestens 100 m/s beträgt, wird die Größe der auf die Haut applizierten Tröpfchen im Vergleich zur WO 2006/040119 A2 deutlich verringert, was für das Eindringen in die Haut von Vorteil ist. Bei Anwendung des erfindungsgemäßen Applikationssystems ergeben sich Tröpfchen mit weniger als 100 nm Durchmesser. Die Tröpfchen können ca. 30 bis 150 µm tief in die Haut eindringen.

Die Austrittsöffnung kann sich 10 bis 30 mm von der Haut entfernt befinden; bis zu einem solchen Abstand ist eine Wirkung nachweisbar. Typischerweise beträgt der Abstand allerdings lediglich 10 bis 20 mm und nicht mehr als 1 cm. Die Strahlbreite beträgt an der Austrittsöffnung 4 bis 6 mm, wobei sich der Strahl nach der Austrittsöffnung kegelförmig ausbreitet. Die Öffnung der Sprühvorrichtung wird bei der Anwendung langsam über die zu behandelnde Hautoberfläche geführt. Die Geschwindigkeit der Bewegung der Düsenöffnung ist aber letztlich unerheblich.

Die Austrittsgeschwindigkeit des Sprühstahls beträgt vorzugsweise mindestens 300 m/s, insbesondere 300 bis 400 m/s. Es hat sich herausgestellt, dass bei einer solchen Strahlgeschwindigkeit die Applikation des Wirkstoffs über die Haut am effektivsten ist. Dies gilt auch dann, wenn als Wirkstoff vergleichsweise große Moleküle verabreicht werden sollen.

Vorzugsweise handelt es sich bei der Sprühvorrichtung um eine Lavaldüse. Bei einer Lavaldüse verengt sich der Querschnitt zunächst und weitet sich zur Austrittsöffnung hin wieder auf, wodurch ein durchströmendes Fluid im Bereich des engsten Querschnitts der Düse auf Überschallgeschwindigkeit beschleunigt werden kann. Mit einer solchen Düse lassen sich somit die gewünschten, hohen Austrittsgeschwindigkeiten erreichen.

Beim Treibgas handelt es sich um Sauerstoff oder ein sauerstoffreiches Gasgemisch mit einem Sauerstoffgehalt von mindestens 60 Vol.-%. Zumeist handelt es sich um weitgehend reinen Sauerstoff mit einem Gehalt von mindestens 98 Vol.-%.

Das Treibgas kann aber neben Sauerstoff auch andere Gase enthalten bzw. dem Treibgas können andere Gase hinzugefügt werden. Es kann sich hierbei um kleinere Verunreinigungen handeln, ebenso gut möglich ist jedoch die Verwendung von Gasen, die eine zusätzliche Wirkung entfalten können. Ein Beispiel für eine Zumischung eines Gases ist die Verwendung von Distickstoffmonoxid N₂O.

Der vom Treibgas erzeugte Überdruck liegt typischerweise in einem Bereich zwischen 1 und 7 bar, vorzugsweise zwischen 2 und 4 bar, besonders bevorzugt bei ca. 3 bar. Auf diese Weise kann eine ausreichend starke Sauerstoffanreicherung in der Zusammensetzung und nachfolgend in der Dermis herbeigeführt werden. Bei erhöhtem Druck ist eine Mikroemulsion in der Lage, erhebliche Mengen Sauerstoff zu lösen, bei einem Druck > 5 bar beispielsweise 10 bis 100mal mehr als bei Atmosphärendruck. Die Eindringtiefe in die Haut liegt typischerweise in einem Bereich von 30 bis 150 µm.

Vorzugsweise ist das Applikationssystem in der Lage, bei Bedarf auch nur das Treibgas, d. h. Sauerstoff oder ein sauerstoffreiches Gasgemisch, ohne die Zusammensetzung auf die Haut zu bringen. Häufig ist es sinnvoll, zunächst die Zusammensetzung auf die Haut zu sprühen und anschließend über einen Zeitraum von mehreren Minuten reinen Sauerstoff zu applizieren, um die Aufnahme des Wirkstoffs und von Sauerstoff durch die Haut weiter zu verbessern.

Besonders bevorzugt ist als versprühbare Zusammensetzung eine Mikroemulsion. Die speziellen Eigenschaften der Mikroemulsionen hängen eng mit ihrem kolloidchemischen Aufbau zusammen. Mikroemulsionen kann man als "kritische Lösung" beschreiben. Diese Systeme besitzen sowohl Eigenschaften, die man von Emulsionen her kennt (partikulär) als auch Eigenschaften, die einer Lösung zukommen (keine Grenzflächenspannung zwischen Öl- und Wasserkomponente, thermodynamische Stabilität). Bei Mikroemulsionen handelt es sich um Emulsionen, bei denen die disperse Phase besonders kleine Tröpfchen bildet, so dass Licht an ihnen nicht gestreut wird. Entsprechend sind Mikroemulsionen, anders als gewöhnliche Emulsionen, nicht milchig trüb sondern klar. Darüber hinaus sind sie dauerhaft stabil. Neben der Öl- und der Wasserphase enthält die Mikroemulsion ein Tensid und ein Cotensid. Die Summe dieser Eigenschaften macht die Mikroemulsionen als Wirkstoff-Trägersystem sehr interessant für den pharmazeutischen und kosmetischen Bereich.

Vorzugsweise beträgt die mittlere Tröpfchengröße der versprühten Zusammensetzung weniger als 300 nm, bevorzugt weniger als 150 nm, weiter bevorzugt weniger als 100 nm. Überraschend hat sich herausgestellt, dass eine Mikroemulsion bei erhöhtem Druck in der Lage ist, erhebliche Mengen Sauerstoff zu lösen, bei einem Druck > 5 bar beispielsweise 500 bis 1.000 mal mehr als bei Atmosphärendruck. Die Menge an Sauerstoff, die in der Mikroemulsion gelöst wird, beträgt typischerweise 0,3 - 0,8 ml O₂/ml Mikroemulsion (bei 25 °C, 3 bar, 25 l/min), dies sind 4 bis 10 mal mehr als gemäß der WO 2006/040119 A2.

Durch die Verwendung einer Sprühvorrichtung mit sehr hoher Austrittsgeschwindigkeit ist es jedoch möglich, auch andere Zusammensetzungen zu applizieren, beispielsweise herkömmliche Emulsionen, Nanoemulsionen, Gele, Mikroschäume, Suspensionen und Zusammensetzungen, die Liposomen oder Mikrobläschen enthalten. Prinzipiell können auch jede einen Wirkstoff gelöst enthaltende Lösung oder beliebige Arten von Dispersionen appliziert werden.

Das Applikationssystem enthält typischerweise einen Vorrat an versprühbarer Zusammensetzung zwischen 0,2 und 10 ml. Im Betrieb beträgt der Volumenfluss an versprühbarer Zusammensetzung typischerweise 0,1 bis 10 ml/min, bevorzugt 0,3 bis 5 ml/min und besonders bevorzugt 0,5 bis 2 ml/min. In der Regel werden pro Applikation somit relativ große Volumina von mindestens 1 ml auf die Haut aufgebracht, wodurch sich das erfindungsgemäße Applikationssystem von der meist für Impfungen verwendeten Jet-Technologie unterscheidet, bei der nur kleine Volumina bis etwa 0,5 ml in die Haut geschossen werden. Typischerweise werden auf 100 cm² Hautoberfläche bis zu 2 ml der Zubereitung gesprüht. Meist schließt sich an die Versprühung der Zusammensetzung noch eine Besprühung der Haut mit dem sauerstoffreichen Treibgas an.

Die Menge an Treibgas, die durch das Applikationssystem pro Minute durch die Austrittsöffnung ausgebracht wird, beträgt 10 bis 100 l, vorzugsweise 15 bis 50 l, besonders bevorzugt 20 bis 30 l. Die Literleistung ist jedoch abhängig vom angewandten Druck. Je nach Größe der Literleistung und des Vorrats an Treibgas ergeben sich somit Standzeiten zwischen 20 und 60 min.

Als Treibgasquelle eignen sich unterschiedliche Vorrichtungen, beispielsweise eine Sauerstoffflasche oder ein Sauerstoffgenerator. Das Applikationssystem kann auch an eine kontinuierlich Sauerstoff fördernde Leitung angeschlossen werden, so dass das System nahezu ununterbrochen betrieben werden kann.

Um die Penetration der Haut weiter zu verbessern, werden die applizierten Tröpfchen zerkleinert. Hierzu wird in Austrittsrichtung des Strahles gesehen hinter der Sprühvorrichtung ein kleines Turbinenrad angeordnet. Dieses Turbinenrad wird durch den Strahl selbst in Rotation versetzt. Möglich ist auch ein zusätzlicher Antrieb des Turbinenrads von außen. Dadurch, dass der Strahl das rotierende Turbinenrad zu passieren hat, wird der Strahl weiter "zerhackt" so dass die auf der Haut auftreffenden Tröpfchen eine sehr geringe Größe aufweisen und zugleich eine größere Hautfläche besprüht wird.

Gemäß einer weiteren vorteilhaften Ausführungsform weist das Applikationssystem Mittel zur temporären Unterbrechung des aus der Sprühvorrichtung austretenden Sprühstrahls auf. Die Unterbrechung sollte so eingestellt sein, dass der Sprühstrahl mindestens einmal, vorzugsweise jedoch mehrmals pro Sekunde unterbrochen wird. Beispielsweise kann der Sprühstrahl 6 bis 14mal, bevorzugt 8 bis 12mal, besonders bevorzugt ca. 10mal pro Sekunde unterbrochen werden. Die Unterbrechung kann bspw. mit Hilfe eines Turbinenrads erfolgen, das den Sprühstrahl "zerhackt". Möglich sind aber auch andere technische Lösungen, um eine Taktung des Sprühstrahls durch kurzzeitige Unterbrechungen herbeizuführen. So sind z. B. andere mechanische Hindernisse denkbar, die kurzzeitig in den Sprühstrahl eingebracht werden. Eine weitere Möglichkeit besteht in der Verwendung von schnell öffnenden Ventilen, die den Fluss des von der Treibgasquelle kommenden Gases steuern. Es handelt sich z. B. um oszillierende oder elektromagnetisch gesteuerte Ventile.

Es hat sich überraschend herausgestellt, dass eine temporäre Unterbrechung des Sprühstrahls nicht nur einen geringeren Gasverbrauch bewirkt, sondern auch für eine bessere Penetration der Haut sorgt, d. h. die gleiche Menge der versprühten Zusammensetzung wird in kürzerer Zeit aufgenommen, ohne dass eine Übersättigung der Haut auftritt. Die Ursachen für diesen überraschenden Effekt sind nicht ganz klar, vermutet wird, dass die Unterbrechung des Sprühstrahls die kinetische Energie der einzelnen Tropfen aufgrund des mit der Unterbrechung einhergehenden Staudrucks erhöht. Ein weiterer möglicher Effekt besteht darin, dass durch die Taktung des Sprühstrahls eine physikalische Stimulation der Haut erfolgt, wodurch die Aufnahmefähigkeit gesteigert wird. Die Kombination aus höherer einwirkender Kraft (Staudruck und Beschleunigungsmoment) und Hautstimulation bewirkt wahrscheinlich eine deutliche Steigerung der Aufnahmefähigkeit der Haut.

Typische medizinische Indikationen für Anwendungen des erfindungsgemäßen Applikationssystems sind Hauterkrankungen mit viralen, bakteriellen und Pilzinfektionen, Hautkrebs, Psoriasis, atopische Dermatitis, entzündliche Haut (Diabetes, Chronisch-venöse Insuffizienz (CVI)) und sonstige Entzündungen, Wundheilung, entzündliche-rheumatische Erkrankungen, Schmerzlinderung/Analgesie und Parodontitis. Auch viele kosmetisch relevante Behandlungen (Cellulite, Vitiligo, Anti-Aging, etc) sind durch Anwendung des Applikationssystem möglich.

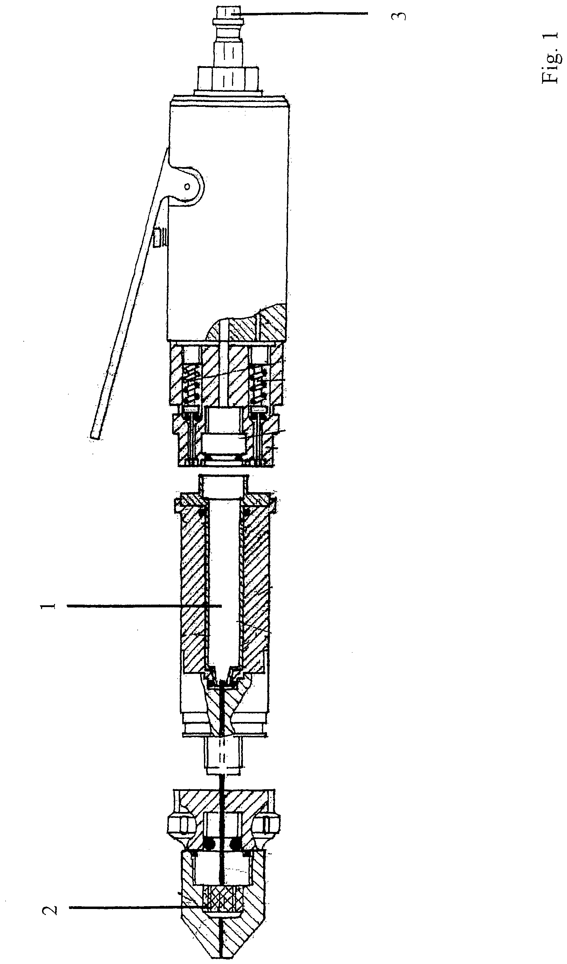

In Figur 1 werden die wesentlichen Teile des erfindungsgemäßen Applikationssystems mit Ausnahme des Turbinenrads dargestellt. Es verfügt über einen Behälter 1 zur Aufnahme der den Wirkstoff enthaltenden versprühbaren Zusammensetzung sowie eine Treibgasquelle 3, hier in Form einer Sauerstoffzuführung über eine Leitung von extern. Das Treibgas treibt die versprühbare Zusammensetzung bei Betätigung des Applikationssystems durch die Sprühvorrichtung 1, bei der es sich um eine Laval-Düse handelt. Behälter 1, Treibgasquelle 3 und Sprühvorrichtung 2 stehen hierfür in geeigneter Weise in Strömungsverbindung.

Die Erfindung wird anhand von Beispielen näher erläutert.

### Beispiel 1: Schmerzlinderung durch Applikation einer Lidocain-haltigen Mikroemulsion mit zusätzlicher Sauerstoff-Begasung

Die Wirkung einer schmerzlindernden 5%-igen Lidocain-haltigen Mikroemulsion (ME-Lido) wurde an 18 freiwilligen Probanden (8 männliche und 10 weibliche Personen im Alter zwischen 23 und 45 Jahren) getestet. Das ME-Lido Präparat wurde im Vergleich zu der EMLA®-Creme (AstraZeneca) untersucht. Es wurden etwa 2 g EMLA®-Creme auf 10 cm² Haut (Unterarm) aufgetragen. Auf ca. 30 cm² Hautareal am Unterarm wurde die ME-Lido-Mikroemulsion mit Hilfe des erfindungsgemäßen Applikationssystems (2 bar, 15 l/min) im Laufe von 60 - 75 Sekunden aufgetragen und anschließend mit Sauerstoff 10 min lang besprüht. Nach unterschiedlichen Einwirkungszeiten wurde das Schmerzempfinden durch kleine Nadelstiche überprüft. Die beteiligten Personen haben ihre Schmerzempfindung auf einer Schmerzgradskala eingeschätzt (0 - kein Schmerz, 5 - normaler Schmerz und 10 - schwerwiegender Schmerz).

**Tabelle 1: Zeit - Analgetisches Potential der EMLA®-Creme und der mit dem erfindungsgemäßen Applikationssystem aufgetragenen Lidocain-haltigen Mikroemulsion.**

| Vehikel | Zeit/min | Schmerzgrad |
|---|---|---|
| ME-Lido mit O₂-Besprühung | 15 | 7,5 |
| | 30 | 5,3 |
| | 45 | 3,0 |
| | 60 | 2,0 |
| | 120 | 2,2 |
| | 180 | 3,0 |
| EMLA®-Creme | 15 | 9,5 |
| | 30 | 9,0 |
| | 45 | 6,0 |
| | 60 | 3,0 |

Das erfindungsgemäße Verfahren zeigt eine moderate Analgesie vor Ablauf von 30 Minuten und eine effektive Analgesie nach 45 Minuten mit anhaltender Wirkung von mindestens 3 Stunden, während EMLA®-Creme erst nach 1 Stunde wirkt.

### Beispiel 2: Antirheumatische Wirkung

Es wurden Behandlungen von an rheumatoider Arthritis leidenden Patienten durchgeführt.

Alle Behandlungen wurden unter Anwendung von Mikroemulsionen durchgeführt, die Diclofenac (5%) und Curcumin (2%) enthielten. Die Mikroemulsion wurde in einer Menge von 0,5 bis 0,7 ml auf die betroffene Stelle mit dem erfindungsgemäßen Applikationssystem aufgetragen und leicht einmassiert. Danach wurde die betroffene Stelle zusätzlich für 20 min mit Sauerstoff begast. Die Behandlungen fanden normalerweise 2- bis 3-mal pro Woche statt. Die Erfolge wurden mit Hilfe der visuellen Analog-Skala (VAS) ermittelt und als Verringerung der Schmerzen erfasst. Die Behandlungsdauer betrug je nach Behandlungserfolg zwischen 1 Monat bis 3 Monaten.

**Tabelle 2: Indikationen, Fallzahl und Behandlungserfolge**

| Indikation | Patientenzahl | erfolgreich | erfolglos |
|---|---|---|---|
| Arthrose im oberen Sprunggelenk | 7 | 6 | 1 |
| Arthritis im Kniegelenk | 7 | 6 | 1 |
| Arthritis in der Hüfte | 7 | 6 | 1 |
| Insgesamt | 21 | 18 | 3 |

Die Tabelle zeigt, dass eine Erfolgsquote von 85,7% erreicht wurde.

### Beispiel 3: Therapie von erkrankten Pferden mit einem entzündlichen Hintergrund.

Alle Behandlungen wurden unter Anwendung einer Boswellsäure (2%) und einer Curcumin-(2%)-haltigen Mikroemulsion durchgeführt. Die Mikroemulsion wurde in einer Menge von 0,5 bis 1,0 ml auf die betroffene Stelle mit dem erfindungsgemäßen Applikationssystem aufgetragen. Danach wurde die betroffene Stelle zusätzlich für 20 min mit Sauerstoff begast. Die Behandlungen fanden normalerweise 2- bis 3-mal pro Woche statt.

**Tabelle 3: Therapie von 15 erkrankten Pferden mit Entzündungen**

| Indikation | Krankheitsfälle | Behandlung erfolgreich | Behandlung erfolglos |
|---|---|---|---|
| Hufgelenksentzündungen | 7 | 6 | 1 |
| Fesselgelenksentzündungen | 8 | 7 | 1 |
| Gesamt | 15 | 13 (87 %) | 2 (13 %) |

Die Tabelle zeigt, dass eine Erfolgsquote von 87 % erreicht wurde.

### Beispiel 4: Behandlung von Cellulite

Für die Behandlung von Probandinnen wurden 0,7 ml Durchblutung fördernde Mikroemulsion (mit Methylnicotinat (1,0 %), Ethylsalicylat (2,0 %) und Carnitin (1,0 %)) mit Hilfe eines Applikationssystems aufgetragen. Die Behandlungen wurden bei einem Sauerstoffdruck von 3 bar und einem Verbrauch von 25 l O₂/min im Laufe von 15 min auf dem ausgewählten Hautareal durchgeführt. Unter diesen Bedingungen wurde die Haut mit Sauerstoff zusätzlich massiert.

Es wurden 28 Probandinnen in einer drei- und fünfwöchigen Kur behandelt. Die Behandlung fand dreimal pro Woche an einer ausgewählten Stelle eines Oberschenkels statt. Die Gegenseite blieb unbehandelt und diente als Kontrolle. Die Probandinnen wurden in zwei Gruppen geteilt. In einer Gruppe fand die Behandlung mit der Mikroemulsion statt, während in der zweiten Gruppe die Probandinnen mit Mikroemulsion plus Sauerstoff behandelt wurden.

Die Hautelastizität wurde mittels Cutometer (DermaLab-System, Cortex Technology, Dänemark) gemessen. Das Verfahren erlaubt die Elastizität der Haut bis zu einer Tiefe von 1,5 mm zu erfassen.

Jeder Wert ist der Mittelwert von drei Messwerten an drei verschiedenen benachbarten Stellen des Hautareals

**Tabelle 4: Relative Zunahme der Hautelastizität (ΔE/E₀) nach 3 und 5 Wochen Behandlung im Vergleich zu unbehandelten Hautstellen.**

| Behandlungsdauer | Behandlung | Hautelastizität, AE/E₀, % |
|---|---|---|
| **3 Wochen** | | |
| **Mikroemulsion** (Gruppe 1) | | |
| | ohne Behandlung | 0,2 ± 0,40 |
| | mit Behandlung | 30,1 ± 2,14 |

| **Mikroemulsion plus Sauerstoff** (Gruppe 2) | | |
|---|---|---|
| | ohne Behandlung | 0,8 ± 0,35 |
| | mit Behandlung | 35,6 ± 2,35 |

| **5 Wochen** | | |
|---|---|---|
| **Mikroemulsion** (Gruppe 1) | | |
| | ohne Behandlung | 0,4 ± 0,33 |
| | mit Behandlung | 41,6 ± 3,16 |

| **Mikroemulsion plus Sauerstoff** (Gruppe 2) | | |
|---|---|---|
| | ohne Behandlung | 0,6 ± 0,45 |
| | mit Behandlung | 52,3 ± 3,36 |

Die Hautelastizität nach 5 Wochen Behandlung ist in Gruppe 1 (Mikroemulsion) um ca. 41 % und in Gruppe 2 (Mikroemulsion plus Sauerstoff) um 52 % gestiegen. Die Behandlung mit Sauerstoff ermöglicht eine Zunahme der Hautelastizität um ca. 10 % im Vergleich zu der Behandlung ohne Sauerstoff.

### Beispiel 5: Behandlung von chronischer Parodontitis

Im Rahmen einer multizentrischen Studie wurden 28 Patienten (Durchschnittsalter war 48 ± 3 Jahre) mit chronischer Parodontitis untersucht.

Die Behandlung begann mit der Initialtherapie. Diese beinhaltete mindestens zwei professionelle Zahnreinigungen, wobei Plaque und Zahnstein manuell und maschinell entfernt wurden. Die für diese Studie notwendigen Untersuchungen erfolgten durch zertifizierte Zahnärzte. Mit einer Parodontalsonde (WHO DB 767) wurden die Sondierungstiefen (ST) und das Attachmentlevel (AL) bestimmt.

Ein Tag nach der letzten Zahnreinigung wurde die adjuvante Therapie mit dem Vehikel (eine O/W-Mikroemulsion, die 2 % Chitooligosaccharide, 0,2 % Curcumin und 0,5 % Ethylsalicylat enthält) mit Hilfe eines erfindungsgemäßen Applikationssystems durchgeführt. Die Begasung mit Sauerstoff erfolgte bei p = 1,7 bar und einem Verbrauch von 10 l/min im Laufe vom 10 Minuten.

In der klinischen Studie wurden 8 Personen ohne adjuvante Therapie (Kontrollgruppe), 10 Patienten mit Mikroemulsion (Gruppe 1) und 10 Patienten mit Mikroemulsion plus Sauerstoffbegasung (Gruppe 2) therapiert. Die Erhebung der klinischen Parameter (ST, AL) erfolgte vor Beginn der Therapie sowie 2 und 4 Monate danach.

Die Durchschnittswerte aller Patienten lagen zum Zeitpunkt der Baseline-Untersuchungen im pathologischen Bereich (ST ≥ 3,5mm und AL ≥ 4,3 mm).

**Tabelle 5.1: Verringerung der ST-Werte (p ≤ 0,001)**

| **Patienten** | **Sondierungstiefe/mm** | | | | |
|---|---|---|---|---|---|
| | **Baseline** | **2 Monate** | **Differenz** | **4 Monate** | **Differenz** |
| Kontrolle | 3,98 ± 0,36 | 2,78 ± 0,35 | 1,2 | 2,72 ± 0,32 | 1,26 |
| Mikroemulsion | 3,96 ± 0,53 | 2,64 ± 0,37 | 1,32 | 2,56 ± 0,27 | 1,40 |
| Mikroemulsion plus O₂ | 4,03 ± 0,68 | 2,45 ± 0,40 | 1,58 | 2,23 ± 0,39 | 1,80 |

Die größte Reduktion erfolgte während der ersten zwei Monate nach Therapie. In den darauf folgenden zwei Monaten wurden bei den ST-Werten innerhalb einer Gruppe nur minimale Unterschiede festgestellt.

Beim Vergleich der Gruppen untereinander wurde in Gruppe 2 (Mikroemulsion plus O₂) eine ausgeprägte Sondierungstiefenreduktion erreicht (1,80 mm nach 4 Monaten). Bei Gruppe 1 (Mikroemulsion) waren die Werte mit 1,40 mm nach 4 Monaten etwas geringer, unterschieden sich jedoch signifikant von denen der Kontrollgruppe.

**Tabelle 5.2: Verringerung der AL-Werte (p ≤ 0,001)**

| **Patienten** | **Attachmentlevel/mm** | | | | |
|---|---|---|---|---|---|
| | **Baseline** | **2 Monate** | **Differenz** | **4 Monate** | **Differenz** |
| Kontrolle | 4,36 ± 0,36 | 3,75 ± 0,35 | 0,61 | 3,60 ± 0,32 | 0,76 |
| Mikroemulsion | 4,27 ± 0,53 | 3,15 ± 0,37 | 1,12 | 3,06 ± 0,27 | 1,27 |
| Mikroemulsion plus O₂ | 4,62 ± 0,68 | 3,11 ± 0,40 | 1,51 | 2,61 ± 0,39 | 2,01 |

Zu jedem Zeitpunkt nach Therapie konnte in Gruppe 2 der größte Attachmentgewinn erreicht werden. Zwei Monate nach Therapie betrug er 1,51 mm, 4 Monate danach 2,00 mm. Die Therapie mit Mikroemulsion (Gruppe 1) erzielte geringere Werte (1,12 mm nach 2 Monaten, 1,27 mm nach 4 Monaten). Der geringste Attachmentgewinn wurde in der Kontrollgruppe gemessen. Der Attachmentgewinn um 0,74 mm (nach 4 Monaten) zwischen der Mikroemulsionsgruppe und der Gruppe mit zusätzlicher Sauerstoffbehandlung deutet auf eine synergistische Wirkung von Mikroemulsion und Sauerstoff hin.

### Beispiel 6: Nachweis der Hautpenetration mittels Fluorescein

Schweineohren wurden ohne Hautverletzungen sofort nach der Schlachtung aus einem Schlachthof abgeholt. Die Ohren wurden gründlich mit milder Seife abgewaschen. Unmittelbar danach wurde die subkutane Fettschicht mit dem Skalpell abgetrennt. Die Haut wurde in einem Container bei - 20°C für weitere Untersuchungen aufbewahrt. Die Trennung der Epidermis von der Dermis erfolgte vorsichtig nach Erwärmung bei 60°C (1 Minute).

Die Durchführung der Penetrationsuntersuchungen erfolgte mit Hilfe einer Diffusionzelle nach Franz mit einer effizienten Diffusionsfläche von 0,636 cm² (SES-Analysensysteme, Deutschland).

0,5 ml Mikroemulsion, die 0,3 mg/ml Fluorescein enthielten, wurden in den Donorteil der Zelle mittels des erfindungsgemäßen Systems (2,0 bar, V = 15,0 l O₂/min) eingegeben. Danach schloss sich eine 15-minütige Sauerstoffbegasung an. Als Akzeptorflüssigkeit wurde isotonische PBS-Lösung verwendet.

Nach 3 Stunden wurden die Häute entnommen und in 4 %iger Formalinlösung fixiert, in Paraffin eingegossen und in 5 µm dicke Scheiben geschnitten und bei - 70°C eingefroren.

Die Hautmuster wurden histologisch untersucht. Die Intensität der Fluoreszenz wurde bei jedem Muster mittels eines konfokalen Laser-Scanning-Mikroskops (Zeiss) mit 10facher Vergrößerung untersucht.

Zwei Regionen (jede 900 Pixel) in der Dermis und der Epidermis wurden ausgewählt, und die Verhältnisse der Intensität in diesen Regionen wurden berechnet. Aus dem generierten Histogramm wurden Intensitäten höher als 150 Pixel angenommen.

**Tabelle 6: Verhältnis der Fluoreszenz Dermis zu Epidermis**

| Präparat | Verhältnis der Fluoreszens in Dermis/Epidermis |
|---|---|
| Fluorescein in Wasser | n.d. |
| Mikroemulsion mit Fluorescein | 0,8 |
| Mikroemulsion mit Fluorescein plus O₂-Begasung | 1,3 |

Wie ersichtlich erfolgt mit dem erfindungsgemäßen Sauerstoffsprüher ein erheblich verbessertes Eindringen in tiefere Hautschichten, so dass der Wirkstoff effektiver wirken kann.

### Beispiel 7: Erhöhung des transkutanen Sauerstoffpartialdrucks (pO₂) mit Hilfe einer Mikroemulsion und Sauerstoff

Die transkutane Sauerstoffpartialdruckmessung ist ein nichtinvasives polarografisches Verfahren zur indirekten Bestimmung des Sauerstoffpartialdrucks (pO₂) an der Hautoberfläche (bis 200 µm Tiefe) und eine indirekte Messung des systemischen arteriellen pO₂. Der Nachweis der Sauerstoffpenetration in die Haut wurde mittels TCM 4 (Radiometer, Dänemark) durch Vermessung des transkutanen Sauerstoffpartialdrucks erfasst.

Zu den Messungen wurden gesunde Probanden ohne Hautverletzungen/Hautkrankheiten ausgewählt. Während der Messungen mussten die Probanden auf Kaffee-, Zigaretten- und Alkoholkonsum verzichten. Die Messungen fanden in einem klimatisierten Raum (T=20-23°C) statt.

Die Haare wurden abrasiert und die Haut wurde mit einem Reinigungsmittel (Bacillol® 25, Fa. Bode Chemie, Deutschland) vorab gereinigt und an der Luft getrocknet.

Die Kleberinge für drei Elektroden wurden an der Innenseite des Unterarmes 3 cm vom Ellbogen (Nr. 1: Kontrolle) und 6 cm vom Ellbogen (Nr. 2: Mikroemulsion) und zwischen den beiden vorgenannten etwas unterhalb ihrer Verbindungslinie (Nr. 3: Mikroemulsion verabreicht über das erfindungsgemäße System mit Sauerstoff) aufgeklebt. Das untersuchte Präparat (0,2 ml O/W-Mikroemulsion) wurde in den Klebering Nr. 2 gegeben und leicht einmassiert bzw. mit Hilfe des erfindungsgemäßen Systems in den Klebering Nr. 3 appliziert. Nach 10 Minuten Wirkung (mit bzw. ohne Sauerstoffbegasung) wurde der Rest der verbliebenen Mikroemulsion vorsichtig mit einem Tuch entfernt. Zu allen Kleberingen wurde Elektrolytlösung (Radiometer, Dänemark) zugegeben und die Elektroden wurden angeschlossen. Die Messung wurde bei 37°C und 45°C durchgeführt. Die Messungen wurden erst nach Erreichen von stabilen Werten (nach ca. 10 Minuten) durchgeführt.

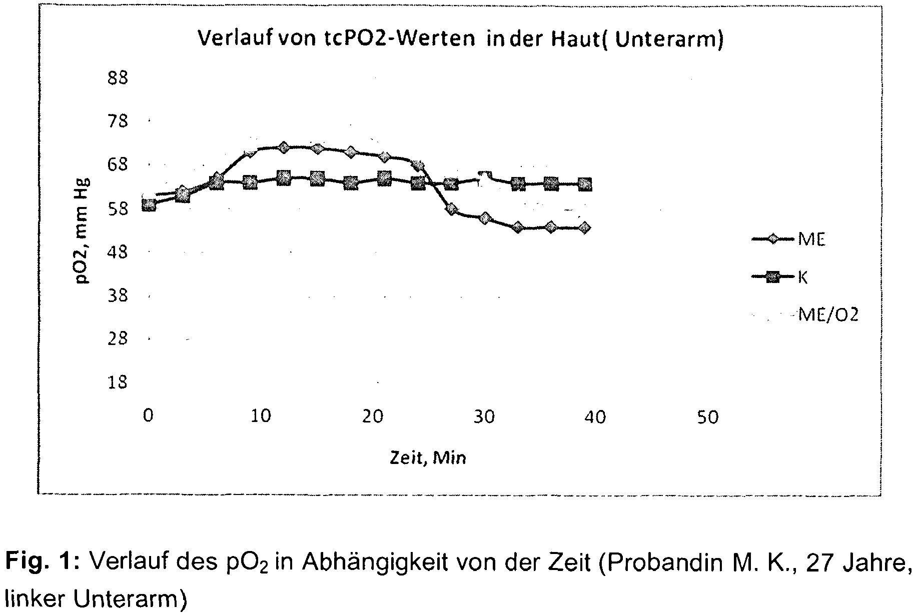

Die mit der Mikroemulsion - sowohl mit als auch ohne Sauerstoffbegasung - behandelte Haut zeigte im Vergleich zu der Kontrolle bereits nach 7 Minuten erhöhte pO₂-Werte in der Haut. Die Werte stiegen im Laufe der Zeit weiter und erreichten nach etwa 15-20 min ihr Maximum (pO₂-Wert der Mikroemulsion um 10 % und pO₂ -Wert Mikroemulsion plus Sauerstoff um 20 % gesteigert im Vergleich zur Kontrolle). Die nach ca. 30 Minuten bei der Mikroemulsion bzw. der Mikroemulsion plus Sauerstoff stärker als bei der Kontrolle abfallenden pO₂-Werte sind ein Zeichen für erhöhten Verbrauch von Sauerstoff in den Hautzellen.

Die geschälte Haut zeigte tcpO₂-Wert von ca. 95 mm Hg, das ist etwa 32 % höher als bei den Kontrollmessungen. Dies spricht dafür, dass das *Stratum corneum* eine wesentliche Barriere für die Sauerstoffpenetration durch die Haut darstellt. Das erfindungsgemäße Verfahren erlaubt die Überwindung dieser Barriere in beachtlichem Ausmaß.

### Beispiel 8: Klinische Studie über eine Psoriasis-Therapie mit Methotrexat-Mikroemulsion ohne und mit Anwendung von Sauerstoff (Synergistische Wirkung)

Zum Nachweis der positiven Effekte bei der Psoriasis-Behandlung unter Anwendung von Methotrexat und des erfindungsgemäßen Applikationssystems auf Abheilung von Psoriasis wurde eine multizentrische placebokontrollierte Parallelgruppen-Studie an 30 Patienten mit der Diagnose des Psoriasis-Plaque-Typs durchgeführt. Die Patientengruppe (männlich/weiblich) war zufallsverteilt mit je 10 Patienten pro Gruppe. Die Behandlung wurde über einen Zeitraum von 14 Wochen durchgeführt mit drei Behandlungen pro Woche. Die PlaceboGruppe erhielt eine entsprechende Placebo-Mikroemulsion ohne Methotrexat. Die Mikroemulsionen wurden direkt (Placebogruppe und Methotrexat-Gruppe) bzw. mit Hilfe des erfindungsgemäßen Applikationssystems (Methotrexat plus Sauerstoff-Gruppe) auf die Haut aufgebracht.

Einschlusskriterien: Die wichtigsten **Einschlusskriterien** lauteten:
- Langzeit-Psoriasis-Plaque-Typ
- Erkrankungsdauer von mindestens 36 Monaten
- Schweregrad der sporadischen Hautveränderungen wurde als leicht (42,0 %) und mittelstark (58 %) beurteilt.
- Es sollten nicht mehr als 25% der Körperoberfläche befallen sein.

Das wichtigste Ausschlusskriterium lautete: Patienten, die eine systemische oder UV-Therapie vor weniger als 2 Monaten haben durchführen lassen.

Das Ausmaß und die Schwere der Hautveränderungen wurden mit Hilfe des *psorisis and severity index* (PASI) ausgewertet. Das Erythem, die Infiltration und die Schuppung an den Körperteilen wurden ausgewertet. Die generelle Einschätzung erfolgte auf einer Skala von 0 bis 5, von komplett clear bis nicht geheilt.

Sowohl die klinische Symptomatik als auch die Aussagen der Patienten wurden dokumentiert. Ferner wurden regelmäßig biochemische Analysen durchgeführt.

Die Wirksamkeit der vorgenommenen Therapie wurde durch Messung der PASI-Werte bestimmt.

Für die Auswertung von Messwerten wurde der Student-t-Test eingesetzt. Die Ergebnisse wurden als p-Wert berechnet. Mit Signifikanzniveau p=0,05 gilt das Ergebnis als statistisch signifikant.

**Tabelle 7: Verbesserung von PASI-Werten abhängig von der Therapiedauer**

| Therapiedauer | Placebo | Verum | |
|---|---|---|---|
| Woche | ME | MTX | MTX plus Sauerstoff |
| 2 | 6,81 ± 0,22 | 5,2 ± 0,24 | 4,1 ± 0,26 |
| 4 | 6,78 ± 0,24 | 4,8 ± 0,31 | 3,0 ± 0,31 |
| 6 | 6,72 ± 0,32 | 4,2 ± 0,27 | 2,8 ± 0,25 |
| 8 | 6,53 ± 0,27 | 3,4 ± 0,25 | 2,4 ± 0,22 |
| 10 | 6,46 ± 0,31 | 3,2 ± 0,22 | 2,0 ± 0,32 |
| 12 | 6,37 ± 0,35 | 2,6 ± 0,27 | 1,7 ± 0,30 |
| 14 | 6,25 ± 0,32 | 2,4 ± 0,23 | 1,5 ± 0,26 |

ME: Mikroemulsion ohne Methotrexat; MTX: Mikroemulsion mit 0,25 Gew.-% Methotrexat

Nach 15-wöchiger Behandlung wurde bei 16 Patienten (80 %) eine komplette Abheilung der psoriatischen Herde beobachtet.

Bei den durchgeführten biochemischen Analysen (Blut: Hb, RBCs, Harnstoff; Leber: SGOT, SGPT, alkalische Phosphatase; Niere: Creatinin, Harnsäure) wurden keine negativen Einflüsse festgestellt. Die Präparate erwiesen sich als gut verträglich. Nur bei 3 Patienten wurde ein leichtes, vorübergehendes, brennendes Gefühl zu Anfang der Behandlung (in der 2. Woche) gemeldet. **Synergistische Wirkung.** Zusätzliche Verabreichung über das erfindungsgemäße System und Behandlung mit Sauerstoff (MTX plus Sauerstoff) beeinflusst die Abheilungsdynamik positiv. Nach 6 Wochen waren die PASI-Werte um 37 % (MTX-Gruppe) bzw. 58 % (MTX plus Sauerstoff) verbessert. Nach 14 Wochen waren die PASI-Werte um 64 % (MTX-Gruppe) bzw. 88 % (MTX plus Sauerstoff) verbessert.

### Beispiel 9: Untersuchung des Penetrationsverhaltens einer Curcumin-haltigen Mikroemulsion mit und ohne Taktung des Sprühstrahls

Das Penetrationsverhalten einer Curcumin-haltigen Mikroemulsion (2%) wurde an 13 freiwilligen Probanden getestet (8 männlich, 5 weiblich, Alter zwischen 26 und 52 Jahren). Die Curcumin-Mikroemulsion wurde mithilfe des erfindungsgemäßen Applikationssystems (2 bar, 15 l/min) auf ca. 25 cm² Haut (Unterarm, 5 x 5 cm Areal) aufgetragen. Es wurde jeweils ein Vergleichsareal gleicher Größe am gegenüberliegenden Unterarm gewählt, welches ebenfalls mit Curcumin-Mikroemulsion in dem erfindungsgemäßen Applikationssystem behandelt wurde (ebenfalls 2 bar und 15 l/min), hier aber in Kombination mit der erfindungsgemäßen Intervalltaktung (Unterbrechung des Sprühstrahls mit einer Frequenz von 10 Hz, dementsprechend 10 Unterbrechungen pro Sekunde und eine dadurch höhere kinetische Energie im unterbrechungsfreien Intervall).

Beide Vergleichsareale wurden mit einer Geschwindigkeit von 1 cm/s und einem Abstand von 1,5 cm von der Haut des Patienten wechselweise in je fünf parallelen Bahnen längs und fünf parallelen Bahnen quer solange behandelt, bis sich auf der Haut ein sichtbarer Ölfilm bildete. Das erfindungsgemäße Applikationssystem gab auf beiden Arealen jeweils 0,0012 ml/s frei.

Während auf dem ohne Unterbrechungstaktung behandelten Areal die Hautsättigung (Bildung eines Ölfilms) durchschnittlich nach ca. 185 Sekunden erreicht war (entsprechend 0,22 ml Curcumin-Mikroemulsion), bildete sich auf dem mit Unterbrechungstaktung behandelten Areal erst nach durchschnittlich ca. 245 Sekunden ein Ölfilm (entsprechend 0,29 ml der Curcumin-Mikroemulsion). Dies entspricht einer durchschnittlichen Erhöhung der Aufnahmefähigkeit der Haut durch die Unterbrechungstaktung um 34%.

Anschließend wurden die Patienten auf einem dritten, ebenfalls 5 x 5 cm großen, benachbarten Vergleichsareal (Unterarm) erneut mit der Curcumin-Mikroemulsion im erfindungsgemäßen Applikationssystem auf einem Arm ohne und auf dem anderen Arm mit Unterbrechungstaktung behandelt, diesmal aber mit einer um 34% gesteigerten Abgabegeschwindigkeit des Applikationssystems (auf 0,0016 ml/s). Alle anderen Parameter blieben unverändert. Erneut wurde kreuzweise in einem 5x5-Gitter behandelt und die Zeit bis zur Bildung eines Ölfilms auf der Haut (Sättigung) gemessen.

Auf dem mit Unterbrechungstaktung behandelten Areal vergingen durchschnittlich 177 Sekunden bis zum Erreichen der Sättigung, entsprechend 0,285 ml, also nahezu der zuvor bei geringerer Geschwindigkeit verabreichten Menge und nahezu in der Zeit, die zuvor bei geringerer Applikationsgeschwindigkeit und ohne Unterbrechungstaktung zu einer Sättigung bereits bei 0,22 ml führte.

Auf dem ohne Unterbrechungstaktung mit 34% erhöhter Applikationsgeschwindigkeit behandelten Areal trat die Sättigung nun nach durchschnittlich 124 Sekunden (entsprechend 0,2 ml) ein.

**Tabelle 8: Zeit bis zum Erreichen der Hautsättigung (Bildung eines Ölfilms)**

| | V = 0,0012 ml/s | | V = 0,0016 ml/s | |
|---|---|---|---|---|
| **Patient** | ohne Taktung | mit Taktung | ohne Taktung | mit Taktung |
| **1** | 180 | 239 | 122 | 177 |
| **2** | 194 | 253 | 129 | 183 |
| **3** | 152 | 203 | 100 | 145 |
| **4** | 168 | 221 | 115 | 159 |
| **5** | 202 | 269 | 136 | 193 |
| **6** | 197 | 258 | 130 | 183 |
| **7** | 184 | 241 | 126 | 180 |
| **8** | 179 | 239 | 121 | 171 |
| **9** | 193 | 257 | 127 | 182 |
| **10** | 196 | 257 | 135 | 196 |
| **11** | 187 | 251 | 128 | 187 |
| **12** | 182 | 238 | 121 | 169 |
| **13** | 191 | 256 | 124 | 176 |
| **Mittelwert** | **185** | **245** | **124** | **177** |

Für die Messungen wurden gesunde Probanden ohne Hautverletzungen/Hautkrankheiten ausgewählt. Die Messungen fanden in einem klimatisierten Raum (T = 20 - 23 °C) statt. Haare wurden abrasiert und die Haut wurde mit einem Reinigungsmittel (Bacillol® 25, Fa. Bode Chemie, Deutschland) vorab gereinigt und an der Luft getrocknet.

Wie ersichtlich ist, fördert die erfindungsgemäße Unterbrechungstaktung in Kombination mit dem erfindungsgemäßen Applikationssystem die Penetration von Substanzen, in diesem Fall Mikroemulsionen, durch die Haut. Die Unterbrechungstaktung ermöglicht demnach eine Steigerung der Anwendungseffizienz um knapp ein Drittel.

## Patentansprüche

1. Applikationssystem zur flächigen, transepidermalen Verabreichung von pharmazeutischen oder kosmetischen Wirkstoffen, mit einem Behälter (1) zur Aufnahme einer einen Wirkstoff enthaltenden, versprühbaren Zusammensetzung, einer Treibgasquelle (3) für ein unter Druck stehendes Gas, das zu mindestens 60 Vol.-% aus Sauerstoff besteht, und einer Sprühvorrichtung (2), wobei Behälter (1), Treibgasquelle (3) und Sprühvorrichtung (2) so miteinander in Strömungsverbindung stehen, dass bei Betätigung der Sprühvorrichtung (2) das Treibgas die versprühbare Zusammensetzung zerstäubt und aus einer Austrittsöffnung der Sprühvorrichtung (2) mit einer Austrittsgeschwindigkeit ≥ 100 m/s in einem kegelförmigen Sprühstrahl heraustreibt,
**dadurch gekennzeichnet, dass** der Öffnungswinkel des Sprühstrahlkegels mindestens 10° beträgt, die Menge an durch die Austrittsöffnung austretendem Treibgas 10 bis 100 l/min beträgt und in Austrittsrichtung des Strahls gesehen hinter der Sprühvorrichtung (2) ein Turbinenrad angeordnet ist.

2. Applikationssystem nach Anspruch 1, **dadurch gekennzeichnet, dass** der Öffnungswinkel des Sprühstrahlkegels mindestens 15° beträgt.

3. Applikationssystem nach Anspruch 1 oder 2, **dadurch gekennzeichnet, dass** die Austrittsgeschwindigkeit mindestens 300 m/s beträgt.

4. Applikationssystem nach einem der Ansprüche 1 bis 3, **dadurch gekennzeichnet, dass** die Sprühvorrichtung (2) eine Lavaldüse ist.

5. Applikationssystem nach einem der Ansprüche 1 bis 4, **dadurch gekennzeichnet, dass** die versprühbare Zusammensetzung eine Mikroemulsion, eine Nanoemulsion, eine Emulsion, ein Gel, ein Mikroschaum, eine Liposomen oder Mikrobläschen enthaltende Zusammensetzung, eine Suspension oder eine Lösung der Wirkstoffe in einem Lösungsmittel ist.

6. Applikationssystem nach einem der Ansprüche 1 bis 5, **dadurch gekennzeichnet, dass** das Volumen der versprühbaren Zusammensetzung im Behälter (1) 0,2 bis 10 ml, bevorzugt 1 bis 10 ml beträgt.

7. Applikationssystem nach einem der Ansprüche 1 bis 6, **dadurch gekennzeichnet, dass** die Menge an durch die Austrittsöffnung austretendem Treibgas 15 bis 50 l/min, bevorzugt 20 bis 30 l/min beträgt.

8. Applikationssystem nach einem der Ansprüche 1 bis 7, **gekennzeichnet durch** Mittel zur temporären Unterbrechung des aus der Sprühvorrichtung (2) herausgetriebenen Sprühstrahls.

9. Applikationssystem nach Anspruch 8, **dadurch gekennzeichnet, dass** der Sprühstrahl mindestens einmal, vorzugsweise mehrmals pro Sekunde unterbrochen wird.

10. Applikationssystem nach Anspruch 9, **dadurch gekennzeichnet, dass** der Sprühstrahl pro Sekunde 6 bis 14mal, bevorzugt 8 bis 12mal, insbesondere ca. 10mal unterbrochen wird.

11. Applikationssystem nach einem der Ansprüche 1 bis 10, **dadurch gekennzeichnet, dass** das Treibgas zu mindestens 98 Vol.-% aus Sauerstoff besteht.

## Claims

1. Application system for the extensive, transepidermal administration of pharmaceutical or cosmetic active substances, comprising a container (1) for accommodating an active substance-containing sprayable composition, a propellant gas source (3) for a pressurized gas that consists of oxygen to an extent of at least 60% by volume, and a spray device (2), wherein the container (1), propellant gas source (3) and spray device (2) are in flow communication with each other such that, on actuation of the spray device (2), the propellant gas atomizes the sprayable composition and forces it out of an exit opening of the spray device (2) with an exit velocity ≥ 100 m/s in a conical spray jet,
**characterized in that** the opening angle of the spray cone is at least 10°, the amount of propellant gas issuing through the exit opening is 10 to 100 1/min and, viewed in the exit direction of the jet, a turbine wheel is arranged behind the spray device (2).

2. Application system according to Claim 1, **characterized in that** the opening angle of the spray cone is at least 15°.

3. Application system according to Claim 1 or 2, **characterized in that** the exit velocity is at least 300 m/s.

4. Application system according to any of Claims 1 to 3, **characterized in that** the spray device (2) is a de Laval nozzle.

5. Application system according to any of Claims 1 to 4, **characterized in that** the sprayable composition is a microemulsion, a nanoemulsion, an emulsion, a gel, a microfoam, a composition comprising liposomes or microbubbles, a suspension, or a solution of the active substances in a solvent.

6. Application system according to any of Claims 1 to 5, **characterized in that** the volume of the sprayable composition in the container (1) is 0.2 to 10 ml, preferably 1 to 10 ml.

7. Application system according to any of Claims 1 to 6, **characterized in that** the amount of propellant gas issuing through the exit opening is 15 to 50 1/min, preferably 20 to 30 1/min.

8. Application system according to any of Claims 1 to 7, **characterized by** a means of temporarily interrupting the spray jet forced out of the spray device (2).

9. Application system according to Claim 8, **characterized in that** the spray jet is interrupted at least once, preferably multiple times, per second.

10. Application system according to Claim 9, **characterized in that** the spray jet is interrupted 6 to 14 times, preferably 8 to 12 times, per second, in particular approx. 10 times.

11. Application system according to any of Claims 1 to 10, **characterized in that** the propellant gas consists of oxygen to an extent of at least 98% by volume.

## Revendications

1. Système d'application destiné à l'administration transépidermique en surface de principes actifs pharmaceutiques ou cosmétiques, muni d'un contenant (1) destiné à la réception d'une composition pulvérisable contenant un principe actif, d'une source de gaz propulseur (3) pour un gaz sous pression, qui est constitué à hauteur d'au moins 60 % en volume par de l'oxygène, et d'un dispositif de pulvérisation (2), le contenant (1), la source de gaz propulseur (3) et le dispositif de pulvérisation (2) étant en communication fluidique les uns avec les autres de telle sorte que, lorsque le dispositif de pulvérisation (2) est actionné, le gaz propulseur atomise la composition pulvérisable et l'expulse par une ouverture de sortie du dispositif de pulvérisation (2) à une vitesse de sortie ≥ 100 m/s en un jet de pulvérisation de forme conique,
**caractérisé en ce que** l'angle d'ouverture du cône du jet de pulvérisation est d'au moins 10°, la quantité de gaz propulseur sortant par l'ouverture de sortie est de 10 à 100 l/min et une roue de turbine est agencée après le dispositif de pulvérisation (2), tel que vu dans la direction de sortie du jet.

2. Système d'application selon la revendication 1, **caractérisé en ce que** l'angle d'ouverture du cône du jet de pulvérisation est d'au moins 15°.

3. Système d'application selon la revendication 1 ou 2, **caractérisé en ce que** la vitesse de sortie est d'au moins 300 m/s.

4. Système d'application selon l'une quelconque des revendications 1 à 3, **caractérisé en ce que** le dispositif de pulvérisation (2) est une tuyère de Laval.

5. Système d'application selon l'une quelconque des revendications 1 à 4, **caractérisé en ce que** la composition pulvérisable est une microémulsion, une nanoémulsion, une émulsion, un gel, une micromousse, une composition contenant des liposomes ou des microbulles, une suspension ou une solution des principes actifs dans un solvant.

6. Système d'application selon l'une quelconque des revendications 1 à 5, **caractérisé en ce que** le volume de la composition pulvérisable dans le contenant (1) est de 0,2 à 10 ml, de préférence de 1 à 10 ml.

7. Système d'application selon l'une quelconque des revendications 1 à 6, **caractérisé en ce que** la quantité de gaz propulseur sortant par l'ouverture de sortie est de 15 à 50 l/min, de préférence de 20 à 30 l/min.

8. Système d'application selon l'une quelconque des revendications 1 à 7, **caractérisé par** des moyens pour l'interruption temporaire du jet de pulvérisation expulsé du dispositif de pulvérisation (2).

9. Système d'application selon la revendication 8, **caractérisé en ce que** le jet de pulvérisation est interrompu au moins une fois, de préférence plusieurs fois par seconde.

10. Système d'application selon la revendication 9, **caractérisé en ce que** le jet de pulvérisation est interrompu par seconde 6 à 14 fois, de préférence 8 à 12 fois, notamment environ 10 fois.

11. Système d'application selon l'une quelconque des revendications 1 à 10, **caractérisé en ce que** le gaz propulser est constitué à hauteur d'au moins 98 % en volume par de l'oxygène.
